**Europäisches Patentamt**

**European Patent Office**

**Office européen des brevets**

(19)

(11) Veröffentlichungsnummer: **0 338 005 B1**

(12)

# EUROPÄISCHE PATENTSCHRIFT

(45) Veröffentlichungstag der Patentschrift:
28.08.91 Patentblatt 91/35

(51) Int. Cl.$^5$: **B23K 26/14**

(21) Anmeldenummer: **88900363.8**

(22) Anmeldetag: **16.12.87**

(86) Internationale Anmeldenummer:
**PCT/EP87/00790**

(87) Internationale Veröffentlichungsnummer:
**WO 88/04592 30.06.88 Gazette 88/14**

(54) **VERFAHREN UND VORRICHTUNG ZUM SCHNEIDEN EINES MATERIALS MITTELS EINES LASERSTRAHLES.**

(30) Priorität: **18.12.86 DE 3643284**

(43) Veröffentlichungstag der Anmeldung:
**25.10.89 Patentblatt 89/43**

(45) Bekanntmachung des Hinweises auf die
Patenterteilung:
**28.08.91 Patentblatt 91/35**

(84) Benannte Vertragsstaaten:
**AT CH DE FR GB IT LI NL**

(56) Entgegenhaltungen:
**GB-A- 2 064 399**
**US-A- 3 843 865**
**US-A- 4 550 240**

(73) Patentinhaber: **AESCULAP AG**
**Möhringer Strasse 125**
**W-7200 Tuttlingen (DE)**

(72) Erfinder: **WROBEL, Walter-Gerhard**
**Stuttgarter Strasse 47**
**W-7200 Tuttlingen (DE)**

(74) Vertreter: **Hoeger, Stellrecht & Partner**
**Uhlandstrasse 14 c**
**W-7000 Stuttgart 1 (DE)**

**Beschreibung**

Die Erfindung betrifft ein Verfahren zum Schneiden eines Materials mittels eines Laserstrahles, der aus einem Lichtleiter austritt und berührungslos auf das zu schneidende Material gerichtet wird.

Laserstrahlung wird in vielfältiger Weise zum Schneiden eines Materials verwendet, beispielsweise bei der Kunststoffbearbeitung oder in biologischen und chirurgischen Verfahren zum Trennen von Gewebe, wobei bei chirurgischen Verfahren neben der Trennung gleichzeitig auch eine Koagulation im Schnittstellenbereich erreicht wird.

Bei all diesen Verfahren besteht die Gefahr einer Verletzung an dem aus dem Laserkopf austretenden Laserstrahl, außerdem können sich Beschädigungen des zu schneidenden Materials im Schnittbereich ergeben. Eine solche Gefährdung kann beispielsweise bei dem aus der US-A-3843865 bekannten Laser eintreten, der im Abstand von der zu bearbeitenden Oberfläche gehalten wird.

Aus der japanischen Offenlegungsschrift 61185260 (= US-A-4676242) ist ein Laser bekannt, bei dem die Strahlung nicht unmittelbar aus einer Glasfaser abgegeben wird, sondern aus einer konisch zulaufenden an dem zu schneidenden Material anliegenden Spitze, in die die Strahlung aus einem Lichtleiter eingekoppelt wird. Die konische Spitze wird von Wasser umspült, um daran haftende Gewebeteilchen zu entfernen. Da aus einer konischen Spitze die Lichtstrahlung unter einem sehr großen Winkel austritt, wird der größte Teil der Strahlung am spitzen Ende der bekannten Anordnung durch die oberflächliche Wasserschicht hindurch abgestrahlt, so daß in der Umgebung der Spitze eine erhebliche Verletzungsgefahr durch Strahlung entsteht, die unter einem Winkel austritt.

Es ist Aufgabe der Erfindung, ein gattungsgemäßes Verfahren derart zu verbessern, daß unbeabsichtigte Verletzungen an der Laserstrahlung sowie eine ungewollte Beschädigung des Materials im Schnittstellenbereich vermieden wird.

Diese Aufgabe wird bei einem Verfahren der eingangs beschriebenen Art erfindungsgemäß dadurch gelöst, daß man den Laserstrahl aus einer senkrecht zur Längsachse eines zylindrischen Lichtleiterkerns verlaufenden Stirnfläche austreten läßt, den Lichtleiterkern im Austrittsbereich allseitig mit einer für die Laserstrahlung durchlässigen Flüssigkeit umgibt, diese Flüssigkeit anschließend an die Stirnfläche zu einem an der Stirnfläche anliegenden kompakten Flüssigkeitsstrahl vereinigt und diesen kompakten Flüssigkeitsstrahl auf das zu schneidende Material richtet, wobei der Abstand zwischen der Stirnfläche und dem zu schneidenden Material so gewählt wird, daß der Flüssigkeitsstrahl bis in den Auftreffbereich auf dem Material kompakt bleibt.

Es hat sich herausgestellt, daß die Laserstrahlung in den sich unmittelbar an das Austrittsende des Lichtleiters anschließenden Flüssigkeitsstrahl eintritt und in diesem nach Art eines Lichtleiters weitgehend verlustfrei geführt wird. Durch den Unterschied der Brechungsindices der Flüssigkeit gegenüber der umgebenden Luft tritt auch hier. Totalreflexion an der Grenzfläche auf, so daß der Flüssigkeitsstrahl die Laserstrahlung bis in den Auftreffbereich des Flüssigkeitsstrahles auf dem zu bearbeitenden Material führt. Dort kann die Laserstrahlung in vollem Umfange wirksam werden. Die Flüssigkeit umspült dabei die Bearbeitungsstelle und kühlt diese. Neben dieser Kühl- und Spülwirkung ist von besonderem Vorteil, daß die Flüssigkeit bei der Materialbearbeitung entstehende Dämpfe und Gerüche binden kann.

Die Laserstrahlung wird bei diesem Verfahren entweder von dem Flüssigkeitsstrahl geführt, teilweise absorbiert und, falls sich dieser noch in Tropfen auflöst, von den Tropfen stark gestreut oder im Gewebe absorbiert. In keinem Fall kann ein freier, gebündelter Laserstrahl unbeabsichtigt austreten und Verletzungen hervorrufen.

Es ist vorteilhaft, wenn man die Flüssigkeitsmenge so wählt, daß der Querschnitt des Flüssigkeitsstrahls stromabwärts des Wellenleiters etwa dem Querschnitt des Wellenleiters entspricht.

Besonders einfach gestaltet sich dieses Verfahren, wenn man als Flüssigkeit Wasser verwendet. Die Extinktionslängen in Wasser betragen bei einer Wellenlänge von 1.06 $\mu$m 90 mm und sind im sichtbaren Bereich noch weit größer.

Durch geeignete Strömungsführung läßt sich ein laminarer, kompakter Flüssigkeitsstrahl erzeugen, der sich bis zu einer Länge von etwa 30 mm nicht in Tropfen auflöst. Man erhält somit einen Flüssigkeitslichtleiter mit einer Länge von etwa 3 cm, so daß der Laserkopf in einem Abstand bis zu 3 cm über dem zu bearbeitenden Werkstoff geführt werden kann.

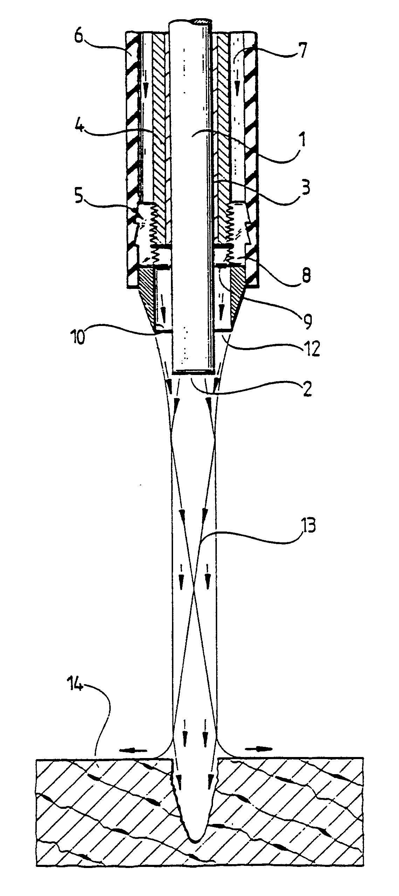

Die nachfolgende Beschreibung einer bevorzugten Ausführungsform der Erfindung dient im Zusammenhang mit der Zeichnung der näheren Erläuterung. Die Zeichnung zeigt eine Schnittdarstellung eines Laserkopfes mit einem sich daran anschließenden Flüssigkeitsstrahl.

Der in der Zeichnung dargestellte Laserkopf umfaßt einen zylindrischen Lichtleiterkern 1, der beispielsweise aus Quarzglas besteht. Er endet an einer senkrecht zur Längsrichtung angeordneten Stirnfläche 2.

Der Lichtleiterkern 1 wird zunächst umgeben von einer Beschichtung 3 (Cladding), die sich ebenso wie ein die Beschichtung 3 umgebender Mantel 4 längs des gesamten Lichtleiterkerns 1 erstreckt, wobei jedoch Mantel 4 und Beschichtung 3 im Bereich vor der Stirnfläche 2 entfernt sind.

Auf das Ende des Mantels 4 ist eine Ringdüse 5 aufgeschraubt, auf die das freie Ende eines Schlauches 6 aufgeschoben ist, der den von Beschichtung 3 und Mantel 4 umgebenen Lichtleiterkern 1 im Abstand umgibt und zwischen dem Mantel 4 und sich selbst einen Ringkanal 7 ausbildet. Die Ringdüse 5 weist an ihrem dem Ringkanal 7 zugewandten Ende mehrere Längsschlitze 8 auf, die sich etwa über die Hälfte der Ringdüsenlänge erstrecken. Über diese Längsschlitze 8 steht der Ringkanal 7 mit dem Innenraum 9 der Ringdüse 5 in Verbindung. Die Wand 10 der Ringdüse 5 umgibt den Lichtleiterkern 1 im Abstand und bildet mit diesem einen Ringspalt 12 aus. Im Bereich dieses Ringspaltes 12 sind Beschichtung 3 und Mantel 4 vom Lichtleiterkern 1 entfernt, so daß der Ringspalt 12 unmittelbar an den Lichtleiterkern 1 angrenzt. Der Lichtleiterkern 1 steht dabei lediglich wenige Millimeter über das stromabwärtige Ende der Ringdüse hervor.

Im Betrieb wird durch den Ringkanal 7 eine Spülflüssigkeit in Richtung der in der Zeichnung angegebenen Pfeile hindurchgeleitet, beispielsweise Wasser. Diese Spülflüssigkeit umgibt den Lichtleiterkern 1 im Bereich des Ringspaltes 12 allseitig und strömt an dem Lichtleiterkern 1 entlang. Im Bereich der Stirnfläche 2 vereinigt sich die ringförmige Strömung zu einem kompakten Flüssigkeitsstrahl 13, der sich unmittelbar an die Stirnfläche 2 anschließt und bei entsprechender Flüssigkeitsdosierung etwa denselben Querschnitt aufweist wie der Lichtleiter 1.

Dieser kompakte Strahl 13 wird auf ein zu schneidendes Material 14 gerichtet.

Durch den Lichtleiterkern 1 über die Stirnfläche 2 austretende Strahlung eines in der Zeichnung nicht dargestellten Lasers gelangt unmittelbar in den sich an den Lichtleiterkern 1 anschließenden Strahl 13 und wird in diesem nach der Art eines Lichtleiters zu der Stelle geführt, an der der Strahl 13 auf das Material 14 auftrifft. In diesem Bereich wird die Laserstrahlung freigesetzt und in dem umgebenden Material absorbiert, so daß dieses Material dadurch geschnitten und bei Verwendung eines biologischen Materials eventuell auch koaguliert wird. Die Auftreffstelle der Laserstrahlung wird durch die Flüssigkeit gleichzeitig gekühlt und gespült, entstehende Dämpfe und Gerüche werden absorbiert.

Wenn als Flüssigkeit Wasser verwendet wird, ergeben sich auch beim Übergang aus dem aus Quarzglas bestehenden Lichleiter in das Wasser praktisch keine Verluste, so daß in dem Wasser-Lichtleiter die Strahlung weitgehend verlustfrei über eine kurze Strecke geführt werden kann, die beispielsweise in der Größenordnung von 3 cm liegt. Diese Länge wird dadurch begrenzt, daß der Strahl nach einer bestimmten Wegstrecke in Tropfen aufgespalten wird. Es tritt dann eine Streuung ein, die die Übertragung eines gerichteten Lichtstrahles unmöglich macht.

Neben den bereits beschriebenen Vorteilen ergibt sich durch den Flüssigkeitsstrahl auch eine gleichzeitige Kühlung des Lichtleiterkerns 1 sowie eine Freispülung desselben, so daß eine Verschmutzung und ein Schmelzen der Stirnfläche 2 nicht eintreten kann.

## Patentansprüche

1. Verfahren zum Schneiden eines Materials (14) mittels eines Laserstrahles, der aus einem Lichtleiter (1) austritt und berührungslos auf das zu schneidende Material (14) gerichtet wird, **dadurch gekennzeichnet,** daß man den Laserstrahl aus einer senkrecht zur Längsachse eines zylindrischen Lichtleiterkerns (1) verlaufenden Stirnfläche (2) austreten läßt, den Lichtleiterkern (1) im Austrittsbereich allseitig mit einer für die Laserstrahlung durchlässigen Flüssigkeit umgibt, diese Flüssigkeit anschließend an die Stirnfläche (2) zu einem an der Stirnfläche (2) anliegenden kompakten Flüssigkeitsstrahl (13) vereinigt und diesen kompakten Flüssigkeitsstrahl (13) auf das zu schneidende Material (14) richtet, wobei der Abstand zwischen der Stirnfläche (2) und dem zu schneidenden Material (14) so gewählt wird, daß der Flüssigkeitsstrahl (13) bis in den Auftreffbereich auf dem Material (14) kompakt bleibt.

2. Verfahren nach Anspruch 1, **dadurch gekennzeichnet,** daß man als Flüssigkeit Wasser oder wässrige Lösungen verwendet.

## Revendications

1. Procédé de découpage d'une matière (14) au moyen d'un rayon laser émergeant d'un guide de lumière (1) et dirigé sans contact sur la matière (14) à découper, caractérisé en ce que l'on fait émerger le rayon laser d'une face frontale (2) s'étendant perpendiculairement à l'axe longitudinal d'une âme cylindrique de guide de lumière (1), en ce que l'on entoure complètement l'âme de guide de lumière (1) dans la zone d'émergence par un liquide transparent au rayonnement laser, en ce qu'à la suite de la face frontale (2) on réunit le liquide en un jet compact de liquide (13) appliqué contre la face frontale (2), et en ce que l'on dirige ce jet compact de liquide (13) sur la matière à découper (14), la distance entre la face frontale (2) et la matière à découper (14) étant choisie de manière à ce que le jet de liquide (13) reste compact jusqu'à la zone d'impact sur la matière (14).

2. Procédé selon la revendication 1, caractérisé en ce que l'on utilise l'eau ou des solutions aqueuses en guise de liquide.

## Claims

1. A process for cutting a material (14) by means of a laser beam which exits from a light guide (1) and is directed in a contactless manner onto the material (14) to be cut, characterized in that the laser beam is made to exit from a front end (2) running perpendicular to the longitudinal axis of a cylindrical light-guide-core (1), the exit region of the light-guide-core (1) is surrounded on all sides with a liquid transmissive with regard to the laser radiation, this liquid is, subsequent to the front end (2), united to form a compact liquid-jet (13) adjacent to the front end (2) and this compact liquid-jet (13) is directed onto the material (14) to be cut, the distance between the front end (2) and the material (14) to be cut being selected in such a manner that the liquid-jet (13) remains compact right into the impact region on the material (14).

2. A process according to Claim 1, characterized in that water or aqueous solutions are used as the liquid.